# EUROPEAN PATENT SPECIFICATION

(11) **EP 4 743 132 B1**
(45) Date of publication and mention of the grant of the patent: **02.12.2026**
(21) Application number: 24746160.1
(22) Date of filing: 09.07.2024
(51) Int. Cl.: A61L 29/16, A61L 29/08, A61L 29/12

(54) **URINARY CATHETER WITH ANTIMICROBIAL AGENTS**
HARNKATHETER MIT ANTIMIKROBIELLEN MITTELN
CATHÉTER URINAIRE PRÉSENTANT DES AGENTS ANTIMICROBIENS

(30) Priority: 10.07.2023 US 202363512709 P
(43) Date of publication of application: 20.05.2026
(73) Proprietor: Hollister Incorporated, Libertyville, IL 60048 (US)
(72) Inventor: SILEIKA, Tadas S., Libertyville, IL 60048 (US); JOCKEL, Mark W., Libertyville, IL 60048 (US); STASIAK, Sandra, Libertyville, IL 60048 (US); SHAH, Ritu, Libertyville, IL 60048 (US)
(74) Representative: FRKelly
(86) International application number: PCT/US2024/037192
(87) International publication number: WO 2025/014940

(56) References cited:
- US-A1- 2009 012 208
- TEIXEIRA-SANTOS RITA ET AL: "Recent advances in antimicrobial surfaces for urinary catheters", CURRENT OPINION IN BIOMEDICAL ENGINEERING, vol. 22, 1 June 2022 (2022-06-01), pages 100394, XP093437541, ISSN: 2468-4511, Retrieved from the Internet <URL:https://pdf.sciencedirectassets.com/315533/1-s2.0-S2468451122X00020/1-s2.0-S2468451122000277/main.pdf?> DOI: 10.1016/j.cobme.2022.100394
- ZHOU NING-LIN ET AL: "A new nanocomposite biomedical material of polymer/Clay-Cts-Ag nanocomposites", CURRENT APPLIED PHYSICS, ELSEVIER, AMSTERDAM, NL, vol. 7, 5 February 2007 (2007-02-05), XP029175652, ISSN: 1567-1739, DOI: 10.1016/J.CAP.2006.11.016
- MARYAM MOOSAVI: "Bentonite Clay as a Natural Remedy: A Brief Review", IRANIAN JOURNAL OF PUBLIC HEALTH, 1 September 2017 (2017-09-01), Iran, pages 1176 - 1183, XP055688533, Retrieved from the Internet <URL:https://www.ncbi.nlm.nih.gov/pmc/articles/PMC5632318/pdf/IJPH-46-1176.pdf>

## Description

The present application claims the benefit of and priority to U.S. Provisional Application No. 63/512,709, filed July 10, 2023.

### FIELD OF THE INVENTION

This disclosure generally relates to a urinary catheter product including antimicrobial agents, wherein the agents are incorporated in a hydration medium of the product. More particularly, this disclosure relates to urinary catheter products with antimicrobial clay(s).

### BACKGROUND

Intermittent catheterization is a good option for many users who suffer from various abnormalities of the urinary system. With the advent of intermittent urinary catheters, individuals with problems associated with the urinary system can conveniently self-catheterize to drain the individual's bladder. Individuals who suffer from urinary incontinence will self-catheterize several times a day.

Although catheters are typically prepared in sterile environments and are provided with safe handling instructions, catheter users are at risk of contracting a urinary tract infection due to several different factors. For example, the catheters may become contaminated during use and introduce bacteria into the urethra. The catheter also may carry bacteria along the urinary tract. US2009012208 discloses a medical device having a wetted hydrophilic coating comprising: a coating composition containing a hydrophilic polymer and a wetting agent comprising water and one or more lubricants. US 2009/012208 A1 discloses a medical device e.g. a urinary catheter having a wetted hydrophilic coating comprising a) a coating composition containing a hydrophilic polymer and b) a wetting agent comprising water and one or more lubricant. The coating compositions may also comprise conventionally used extenders or fillers such as polysaccharides like CMC, antioxidants, fibres, salts, clay, buffers, or pigments in an amount up to 30% of the total weight of the coating composition prepared. The medical device is suitably stored in a package which may contain additional water, wetting agent comprising lubricant or another aqueous solution (e.g. saline).

Therefore, there is a need for methods and devices that allow catheter users to prevent urinary tract infections.

### SUMMARY OF INVENTION

The scope of protection is defined by the claims.

In one aspect, a urinary catheter product according to claims 1-8 is provided.

In yet another aspect, a method of making an antimicrobial catheter according to claims 11-15 is provided. coating.

### BRIEF DESCRIPTION OF DRAWINGS

FIG. 1 is a plan view of an embodiment of the urinary catheter product.
FIG. 2 is a side view of a portion of the catheter and an enlarged inset of the catheter outer surface of the urinary catheter product of FIG. 1.
FIG. 3 is a plan view of another embodiment of the urinary catheter product.
FIG. 4 is a cross-sectional view of the urinary catheter product of FIG. 3.

### DETAILED DESCRIPTION

A more detailed description of the device in accordance with the present disclosure is set forth below.

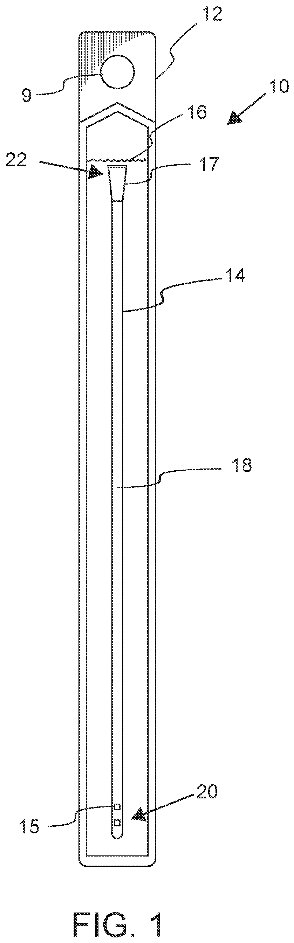

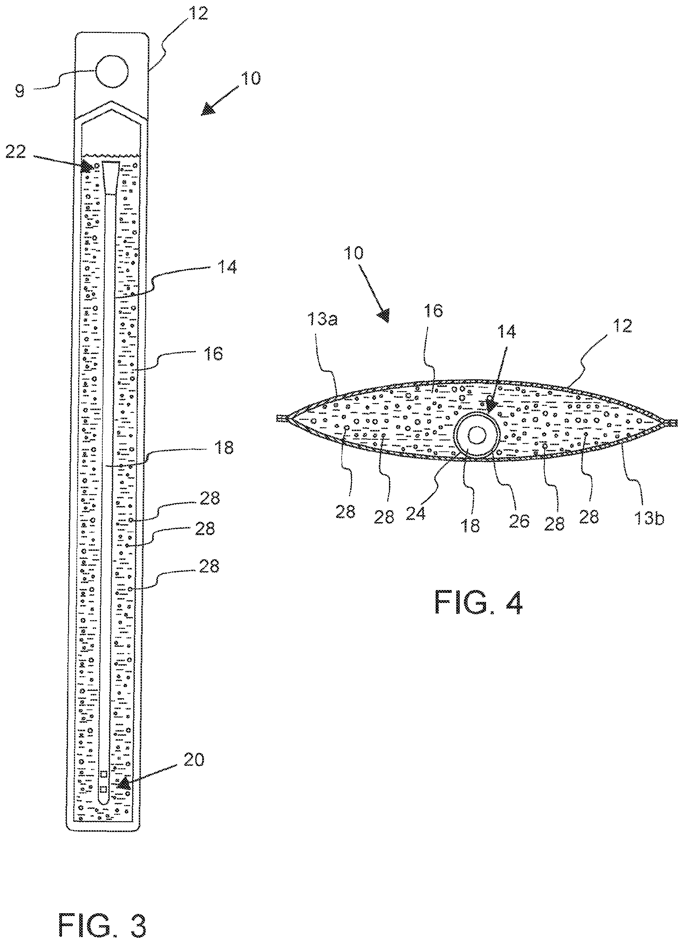

FIGS. 1 and 3 show a urinary catheter product 10. The catheter product 10 includes a package 12 containing a urinary catheter 14 and a hydration medium 16. The package includes a cavity configured to receive the urinary catheter 14 and the hydration medium 16. In one embodiment the package 12 may be made of first and second opposing sheets 13a and 13b (shown in FIG. 4). The sheets may be sealed along the edges, to define the cavity. In some embodiments, the package may have a gripping member 9 to assist the user in holding the package 12. In some embodiments, the package 12 may have a tear strip (not shown) to assist the user in opening the package 12. The package may have a generally rectangular shape. Other packages known in the art configured to contain a catheter and hydration medium may be used without departing from the scope of the disclosure.

FIG. 1 further shows urinary catheter 14 inside the package 12. Urinary catheter 14 includes a catheter tube 18 having a proximal insertion end 20 and a distal drainage end 22. A lumen extends from the proximal insertion end 20 to the distal drainage end 22. The proximal insertion end 20 includes at least one opening 15 for receiving urine in communication with the lumen. In one embodiment, the at least one opening 15 for receiving urine may be an eyelet. In another embodiment, the at least one opening 15 for receiving urine may be multiple eyelets. The at least one opening 15 for receiving urine may vary in shape and size without departing from the scope of the disclosure.

Furthermore, the distal drainage end 22 of the catheter tube 18 includes a drainage opening for draining urine from the lumen. In one embodiment, the distal drainage end 22 includes a drainage member 17. The drainage member 17 may be, for example, but not limited to, a funnel or connector for connection to a drainage bag (not shown). Other drainage members known in the art may be used without departing from the scope of the disclosure.

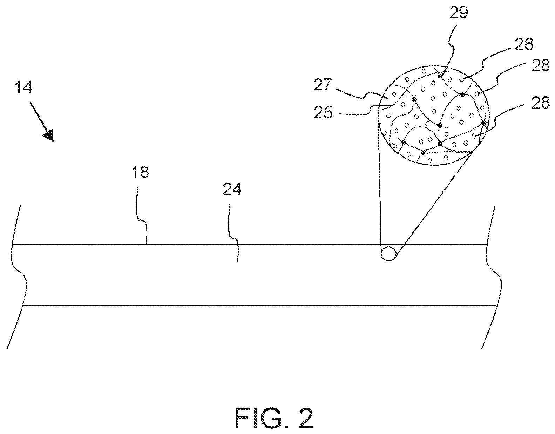

The urinary catheter tube 18 includes a hydrophilic polymer coating 24 (shown in FIGS. 2 and 4) on an outer surface 26 of the catheter tube 18. The hydrophilic polymer coating 24 is configured to absorb a hydration medium 16 to lubricate the catheter to aid in insertion into a urethra.

The hydrophilic coating solution may include a suitable solvent (such as water and/or ethanol), hydrophilic polymer(s) and optional additives. The resulting hydrophilic coating may include any suitable hydrophilic polymer, such as polyvinylpyrrolidone, polyethylene oxide or polyvinyl alcohol. Examples of hydrophilic polymer solutions used to create a hydrophilic polymer coating and hydrophilic coatings are disclosed in WO2016/168461, filed April 14, 2016, to Hollister. Such hydrophilic coating solutions may include a solvent, hydrophilic polymer(s), other monomers/oligomers, and optional additives. In one alternative, the hydrophilic coatings may include a hydrophilic polymer and polyethylene glycol diacrylate having a number average molecular weight of less than about 1000. In one embodiment the number average molecular weight may be less than about 600. In one alternative, the hydrophilic coating comprises about 80 wt% to about 99.5 wt% hydrophilic polymer and about 0.5 wt% to about 20 wt% polyethylene glycol diacrylate. Other hydrophilic polymer solutions and coatings may be used without departing from the scope of the disclosure. During the curing process, the hydrophilic polymer solution crosslinks to create a hydrophilic polymer coating 24 containing a polymer matrix 25 with interstices 27 (shown in FIG. 2).

An antimicrobial clay is located in the package 12 of the urinary catheter product 10. The antimicrobial clay may control microbial proliferation and attachment and thus aids in the reduction of urinary tract infections. For example, some clays have been demonstrated to have antimicrobial properties against many Gram-negative and Gram-positive bacteria and yeast, which also tend to be implicated in the bacteriology of urinary tract infections.

Certain clays may possess antimicrobial properties due to several factors, including their physical and chemical properties. For instance, some clays include antimicrobial metal ions such as, but not limited to, iron (Fe), copper (Cu), and zinc (Zn). The antimicrobial metal ions in the clay(s) may interact with the proteins in bacteria or other microorganisms, disrupting their normal function and leading to their death. The toxicity of metal ions can vary depending on the specific ion and its concentration. Also, some clays, particularly those that contain elements like iron, can catalyze reactions that produce reactive oxygen species. These highly reactive chemicals can damage microbial cells, disrupting their functions and causing cell death.

Additionally, some clays can influence the pH of their surrounding environment, making it more acidic or alkaline. Extreme pH environments can be detrimental to microbial survival. For example, bacteria generally prefer a neutral pH (about 7) and can be killed or have their growth inhibited in highly acidic or alkaline conditions.

Absorption and adsorption properties of some clays may also contribute to their antimicrobial nature. For example, some clays have a high surface area and can absorb or adsorb various substances, including water, organic matter, and microbes. When microbes are absorbed or adsorbed, they can be trapped within the clay matrix, physically separating them from their environment and inhibiting their growth or even killing them directly.

Furthermore, particle size and shape may also contribute to the antimicrobial nature of certain clays. The small particle size of certain clays can physically damage microbial cells. Additionally, the sharp edges of clay particles can pierce the cell membranes of bacteria, leading to cell lysis and death.

Antimicrobial clays included in the catheter product 10 may be bentonite, montmorillonite, illite, kaolin, or any combination of such clays. Additionally, other antimicrobial clays known in the art may be used without departing from the scope of the disclosure.

FIG. 2 (not according to the claimed invention) shows a portion of the catheter tube 18 with an enlarged inset showing the hydrophilic polymer coating 24 with antimicrobial clay granules incorporated into the coating 24. During curing, the hydrophilic polymer solution crosslinks 29 to create a hydrophilic polymer coating 24 with a polymer matrix 25 and interstices 27. Without being chemically bonded to the coating, the clay granules may be located in the interstices 27 of the polymer matrix 25. The antimicrobial clay in the coating may be in a concentration from about 0.001 wt.% to about 10 wt.%, and preferably in a concentration from about 0.5 wt.% to about 5 wt.% depending on desired efficacy. In an embodiment, the antimicrobial clay in the coating may be in a concentration of about 1 wt.%.

After coating the catheter tube 18 and before use, the hydrophilic polymer coating may be hydrated with a hydration medium, including, but not limited to, a hydrating vapor and/or liquid. After the hydrophilic polymer coating has been hydrated, the concentration of the antimicrobial clay in the hydration medium with the hydrophilic coating may be in a range of 14 µg/mL to 28 µg/mL, and preferably in a range of 14 µg/mL to 24 µg/mL. In an alternative, the antimicrobial clay may be in a concentration of at least 14 µg/mL. In another alternative, the antimicrobial clay may be in a concentration of at least 24 µg/mL, and preferably at least 28 µg/mL. Higher concentrations of antimicrobial clay are more bacteriostatic and potentially bactericidal.

In another embodiment, the antimicrobial clay may be incorporated in the hydration medium 16. FIGS. 3 and 4 show a urinary catheter product 10 including a package 12 containing a urinary catheter 14 and a hydration medium 16. The hydration medium may be a liquid, including water or an aqueous solution. For example, the hydration medium may be sterile water, saline solution, or another hydration medium containing different solutes. In particular, saline solution may be used for catheters that will be inserted into areas of the body where the natural fluid is saline-like (e.g., the urinary tract).

In an embodiment, the antimicrobial clay incorporated in the hydration medium 16 may be bentonite, montmorillonite, illite, kaolin, or any combination of such clays. In an embodiment, the antimicrobial clay may be a clay including a metal ion such as iron (Fe), copper (Cu), and/or zinc (Zn). In an embodiment, the antimicrobial clay may catalyze reactions that produce reactive oxygen species. In an embodiment, the antimicrobial clay may influence the pH of the surrounding environment, for example, the antimicrobial clay may make its environment more acidic or alkaline. In an embodiment, the antimicrobial clay may have a high surface area and can absorb or adsorb various substances, including water, organic matter, and microbes. In an embodiment, the antimicrobial clay may include a small particle size that can physically damage microbial cells and/or sharp edges that can pierce the cell membranes of bacteria. In an embodiment, the antimicrobial clay may be a clay including any or a combination of the properties described above (i.e., the clay may include a metal ion, produce reactive oxygen species, influence pH, have a high surface area, include a small particle size, and/or include sharp edges). It shall be understood that the antimicrobial clay incorporated into the hydration medium 16 may be any of the clays or a combination of clays described herein.

The antimicrobial clay may be suspended or solvated in the hydration liquid. In one alternative, the hydration medium is configured to deposit the antimicrobial within the hydrophilic polymer coating 24. After incorporating the antimicrobial clay into the hydration liquid, the antimicrobial clay is deposited into the hydrophilic polymer coating by hydrating the coating with the liquid. For instance, the hydration liquid carries the antimicrobial clay incorporated in hydration medium 16 into the hydrophilic coating 24. In one embodiment, the hydration medium 16 carries the antimicrobial clay into the matrix of the hydrophilic polymer coating 24. For example, the hydration medium 16 carries the antimicrobial clay into the interstices 27 of the matrix 25. In an embodiment, the hydration medium 16 including antimicrobial clay may be applied to the hydrophilic polymer coating prior to sterilization.

The hydration medium may include the antimicrobial clay in a concentration range of 14 µg/mL to 28 µg/mL,and preferably in a range of 14 µg/mL to 24 µg/mL. In an alternative, the antimicrobial clay may be in a concentration of at least 14 µg/mL. In another alternative, the antimicrobial clay may be in a concentration of at least 24 µg/mL, and preferably at least 28 µg/mL. Higher concentrations of antimicrobial clay are more bacteriostatic and potentially bactericidal.

In yet another embodiment, the antimicrobial clay is incorporated into both the hydrophilic polymer coating 24 and the hydration medium 16 to enhance any antimicrobial efficacy.

## Claims

1. A urinary catheter product comprising:
a package containing a urinary catheter, the urinary catheter comprising:
a catheter tube having a proximal insertion end and a distal drainage end;
a hydrophilic polymer coating on an outer surface of the catheter tube; and an antimicrobial clay inside the package
a hydration medium inside the package, wherein the hydrophilic polymer coating is hydrated by the hydration medium, and wherein the hydration medium comprises a hydration liquid and the antimicrobial clay is incorporated into the hydration liquid.

2. The urinary catheter product of claim 1, wherein the antimicrobial clay is incorporated into the hydrophilic polymer coating.

3. The urinary catheter product of claim 2, wherein the clay is located within a matrix of the hydrophilic polymer coating.

4. The urinary catheter product of any one of claims 1-3, wherein the hydration liquid is configured to deposit the antimicrobial clay into the hydrophilic polymer coating.

5. The urinary catheter product of any one of claims 1-4, wherein the hydration liquid includes the antimicrobial clay in a concentration of at least 14 µg/mL.

6. The urinary catheter product of any one of claims 1-5, wherein the hydration liquid includes the antimicrobial clay in a concentration of at least 28 µg/mL, preferably at least 24 µg/mL.

7. The urinary catheter product of any one of claims 1-6, wherein the clay is bentonite, montmorillonite, illite, kaolin, or any combination of such clays.

8. The urinary catheter product of any one of claims 1-7, wherein the antimicrobial clay comprises grains with a grain size not larger than about 0.004 mm, and preferably in a range of about 5 nm to about 0.004 mm.

9. A method for making an antimicrobial catheter comprising:
providing a urinary catheter comprising:
a catheter tube having a proximal insertion end and a distal drainage end; and
a hydrophilic polymer coating on an outer surface of the catheter tube;
hydrating the hydrophilic polymer coating with a hydration medium, wherein the hydration medium comprises a hydration liquid and an antimicrobial clay is incorporated in the hydration liquid; and
adding an antimicrobial clay to the hydrophilic polymer coating.

10. The method of claim 9, comprising incorporating the antimicrobial clay to the hydrophilic polymer coating.

11. The method of any one of claims 9-10, wherein the hydration liquid deposits the antimicrobial clay into the hydrophilic polymer coating.

12. The method of any one of claims 9-11, wherein the hydration liquid includes the antimicrobial clay in a concentration of at least 14 µg/mL.

13. The method of any one of claims 9-12, wherein the hydration liquid includes the antimicrobial clay incorporated in the hydrophilic polymer coating in a concentration of at least 28 µg/mL, preferably at least 24 µg/mL.

14. The method of any one of claims 9-13, wherein the clay is bentonite, montmorillonite, illite, kaolin, or any combination of such clays.

15. The method of any one of claims 9-14, wherein the antimicrobial clay comprises grains with a grain size not larger than about 0.004 mm, and preferably in a range of about 5 nm to about 0.004 mm.

## Patentansprüche

1. Harnkatheterprodukt, umfassend:
eine Verpackung, die einen Harnkatheter enthält, wobei der Harnkatheter Folgendes umfasst:
einen Katheterschlauch, der ein proximales Einführende und ein distales Abflussende aufweist;
eine hydrophile Polymerbeschichtung auf einer Außenfläche des Katheterschlauchs; und
einen antimikrobiellen Ton im Inneren der Verpackung ein Hydratisierungsmedium im Inneren der Verpackung, wobei die hydrophile Polymerbeschichtung durch das Hydratisierungsmedium hydratisiert wird, und wobei das Hydratisierungsmedium eine Hydratisierungsflüssigkeit umfasst und der antimikrobielle Ton in die Hydratisierungsflüssigkeit eingearbeitet ist.

2. Harnkatheterprodukt nach Anspruch 1, wobei der antimikrobielle Ton in die hydrophile Polymerbeschichtung eingearbeitet ist.

3. Harnkatheterprodukt nach Anspruch 2, wobei der Ton in einer Matrix der hydrophilen Polymerbeschichtung angeordnet ist.

4. Harnkatheterprodukt nach einem der Ansprüche 1-3, wobei die Hydratisierungsflüssigkeit dafür konfiguriert ist, den antimikrobiellen Ton in die hydrophile Polymerbeschichtung abzulagern.

5. Harnkatheterprodukt nach einem der Ansprüche 1-4, wobei die Hydratisierungsflüssigkeit den antimikrobiellen Ton in einer Konzentration von mindestens 14 µg/mL enthält.

6. Harnkatheterprodukt nach einem der Ansprüche 1-5, wobei die Hydratisierungsflüssigkeit den antimikrobiellen Ton in einer Konzentration von mindestens 28 µg/mL enthält, vorzugsweise mindestens 24 µg/mL.

7. Harnkatheterprodukt nach einem der Ansprüche 1-6, wobei der Ton Bentonit, Montmorillonit, Illit, Kaolin oder eine beliebige Kombination solcher Tone ist.

8. Harnkatheterprodukt nach einem der Ansprüche 1-7, wobei der antimikrobielle Ton Körner mit einer Korngröße umfasst, die nicht größer als etwa 0,004 mm ist, und vorzugsweise in einem Bereich von etwa 5 nm bis etwa 0,004 mm.

9. Verfahren zum Herstellen eines antimikrobiellen Katheters, umfassend:
Bereitstellen eines Harnkatheters, umfassend:
einen Katheterschlauch, der ein proximales Einführende und ein distales Abflussende aufweist; und
eine hydrophile Polymerbeschichtung auf einer Außenfläche des Katheterschlauchs;
Hydratisieren der hydrophilen Polymerbeschichtung mit einem Hydratisierungsmedium, wobei das Hydratisierungsmedium eine Hydratisierungsflüssigkeit umfasst und ein antimikrobieller Ton in die Hydratisierungsflüssigkeit eingearbeitet ist; und
Hinzufügen eines antimikrobiellen Tons zu der hydrophilen Polymerbeschichtung.

10. Verfahren nach Anspruch 9, umfassend ein Einarbeiten des antimikrobiellen Tons in die hydrophile Polymerbeschichtung.

11. Verfahren nach einem der Ansprüche 9-10, wobei die Hydratisierungsflüssigkeit den antimikrobiellen Ton in die hydrophile Polymerbeschichtung ablagert.

12. Verfahren nach einem der Ansprüche 9-11, wobei die Hydratisierungsflüssigkeit den antimikrobiellen Ton in einer Konzentration von mindestens 14 µg/mL enthält.

13. Verfahren nach einem der Ansprüche 9-12, wobei die Hydratisierungsflüssigkeit den in die hydrophile Polymerbeschichtung eingearbeiteten antimikrobiellen Ton in einer Konzentration von mindestens 28 µg/mL enthält, vorzugsweise mindestens 24 µg/mL.

14. Verfahren nach einem der Ansprüche 9-13, wobei der Ton Bentonit, Montmorillonit, Illit, Kaolin oder eine beliebige Kombination solcher Tone ist.

15. Verfahren nach einem der Ansprüche 9-14, wobei der antimikrobielle Ton Körner mit einer Korngröße umfasst, die nicht größer als etwa 0,004 mm ist, und vorzugsweise in einem Bereich von etwa 5 nm bis etwa 0,004 mm.

## Revendications

1. Produit de cathéter urinaire comprenant :
un emballage contenant une sonde urinaire, la sonde urinaire comprenant :
un tube de cathéter ayant une extrémité d'insertion proximale et une extrémité de drainage distale ;
un revêtement polymère hydrophile sur une surface extérieure du tube de cathéter ; et
une argile antimicrobienne à l'intérieur de l'emballage un milieu d'hydratation à l'intérieur de l'emballage, dans lequel le revêtement polymère hydrophile est hydraté par le milieu d'hydratation, et dans lequel le milieu d'hydratation comprend un liquide d'hydratation et l'argile antimicrobienne est incorporée dans le liquide d'hydratation.

2. Produit de cathéter urinaire selon la revendication 1, dans lequel l'argile antimicrobienne est incorporée dans le revêtement polymère hydrophile.

3. Produit de cathéter urinaire selon la revendication 2, dans lequel l'argile est située au sein d'une matrice du revêtement polymère hydrophile.

4. Produit de cathéter urinaire selon l'une quelconque des revendications 1 à 3, dans lequel le liquide d'hydratation est configuré pour déposer l'argile antimicrobienne dans le revêtement polymère hydrophile.

5. Produit de cathéter urinaire selon l'une quelconque des revendications 1 à 4, dans lequel le liquide d'hydratation comporte l'argile antimicrobienne à une concentration d'au moins 14 µg/mL.

6. Produit de cathéter urinaire selon l'une quelconque des revendications 1 à 5, dans lequel le liquide d'hydratation comporte l'argile antimicrobienne à une concentration d'au moins 28 µg/mL,de préférence d'au moins 24 µg/mL.

7. Produit de cathéter urinaire selon l'une quelconque des revendications 1 à 6, dans lequel l'argile est de la bentonite, de la montmorillonite, de l'illite, du kaolin ou une quelconque combinaison de ces argiles.

8. Produit de cathéter urinaire selon l'une quelconque des revendications 1 à 7, dans lequel l'argile antimicrobienne comprend des grains ayant une granulométrie ne dépassant pas environ 0,004 mm, et de préférence comprise dans une plage entre environ 5 nm et environ 0,004 mm.

9. Procédé de fabrication d'un cathéter antimicrobien comprenant :
la fourniture d'un cathéter urinaire comprenant :
un tube de cathéter ayant une extrémité d'insertion proximale et une extrémité de drainage distale ; et
un revêtement polymère hydrophile sur une surface extérieure du tube de cathéter ;
l'hydratation du revêtement polymère hydrophile avec un milieu d'hydratation, dans lequel le milieu d'hydratation comprend un liquide d'hydratation et une argile antimicrobienne est incorporée au liquide d'hydratation ; et
l'ajout d'une argile antimicrobienne au revêtement polymère hydrophile.

10. Procédé selon la revendication 9, comprenant l'incorporation de l'argile antimicrobienne au revêtement polymère hydrophile.

11. Procédé selon l'une quelconque des revendications 9 et 10, dans lequel le liquide d'hydratation dépose l'argile antimicrobienne dans le revêtement polymère hydrophile.

12. Procédé selon l'une quelconque des revendications 9 à 11, dans lequel le liquide d'hydratation comporte l'argile antimicrobienne à une concentration d'au moins 14 µg/mL.

13. Procédé selon l'une quelconque des revendications 9 à 12, dans lequel le liquide d'hydratation comporte l'argile antimicrobienne incorporée dans le revêtement polymère hydrophile à une concentration d'au moins 28 µg/mL, de préférence d'au moins 24 µg/mL.

14. Procédé selon l'une quelconque des revendications 9 à 13, dans lequel l'argile est de la bentonite, de la montmorillonite, de l'illite, du kaolin ou une quelconque combinaison de ces argiles.

15. Procédé selon l'une quelconque des revendications 9 à 14, dans lequel l'argile antimicrobienne comprend des grains ayant une granulométrie ne dépassant pas environ 0,004 mm, et de préférence comprise dans une plage entre environ 5 nm et environ 0,004 mm.
